(19) Europäisches Patentamt
European Patent Office
Office européen des brevets

(11) Veröffentlichungsnummer: **0 037 471**
**B1**

(12) # EUROPÄISCHE PATENTSCHRIFT

(45) Veröffentlichungstag der Patentschrift: **30.05.84**

(21) Anmeldenummer: **81101779.7**

(22) Anmeldetag: **11.03.81**

(51) Int. Cl.³: **C 07 D 403/06**, C 07 D 401/06, C 07 D 405/06, C 07 D 409/06, C 07 D 413/06, C 07 D 417/06, C 07 D 233/64, A 61 K 31/33, A 61 K 31/415

(54) **1-Aroyl-2-phenylamino-2-imidazoline, ihre Herstellung und diese enthaltende Arzneimittel.**

(30) Priorität: **24.03.80 DE 3011327**

(43) Veröffentlichungstag der Anmeldung: **14.10.81 Patentblatt 81/41**

(45) Bekanntmachung des Hinweises auf die Patenterteilung: **30.05.84 Patentblatt 84/22**

(84) Benannte Vertragsstaaten: **AT BE CH DE FR GB IT LI LU NL SE**

(56) Entgegenhaltungen:
**DE - A - 2 505 297**
**DE - A - 2 652 004**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **May, Hans-Joachim, Dr., Birkenweg 40, D-6730 Neustadt (DE)**
Erfinder: **Lenke, Dieter, Dr., Kekuleplatz 1, D-6700 Ludwigshafen (DE)**
Erfinder: **Gries, Josef, Dr., Roemerweg 43, D-6706 Wachenheim (DE)**
Erfinder: **Teschendorf, Hans-Juergen, Dr., Rene-Bohn-Strasse 4, D-6700 Ludwigshafen (DE)**
Erfinder: **Worstmann, Wolfgang, Dr., Franz-Marc-Weg 70, D-4400 Hiltrup (DE)**

**EP 0 037 471 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 1-Aroyl-2-phenylamino-2-imidazoline, Verfahren zu deren Herstellung, Arzneimittel, welche die neuen Substanzen enthalten, sowie deren Verwendung bei der Behandlung der Hypertonie, Migräne und der koronaren Herzkrankheit.

Aus der DE-AS Nr. 2559711 ist es bekannt, dass Benclonidin (1-Benzoyl-2-(2',6'-dichlorphenylamino)-2-imidazolin), ebenso wie das Clonidin (2-(2',6'-Dichlorphenylamino)-2-imidazolin), blutdrucksenkende Eigenschaften aufweist. Erstere Verbindung soll geringere sedative Nebenwirkungen besitzen als die Grundverbindung Clonidin und darüber hinaus bei oraler Gabe gut resorbiert werden.

Weiterhin werden in der DE-OS Nr. 2505297 neben dem 1-Benzoylderivat des Clonidins auch die drei isomeren Toluoylderivate beschrieben, die ein ähnlich gutes Verhältnis zwischen blutdrucksenkender und sedativer Wirkung haben sollen.

Es wurden nun Verbindungen mit günstigeren Eigenschaften gefunden.

Gegenstand der Erfindung sind 1-Aroyl-2-phenylamino-2-imidazoline der allgemeinen Formel I:

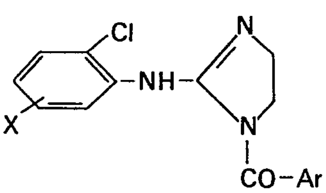

(I)

worin

X ein Chloratom in der 3- oder 6-Stellung des Phenylringes, und

Ar einen 2-Chinolinyl-, 6-Fluor-2-chinolinyl-, 2-Furyl-, 3-Pyridyl- oder 4-Pyridylrest darstellen,

sowie deren Salze mit physiologisch verträglichen Säuren.

Bevorzugt sind die 2,6-Dichlorverbindungen.

Von den für Ar genannten Bedeutungen sind die folgenden Reste bevorzugt: 3-Pyridyl und 2-Chinolinyl.

Gegenstand der Erfindung ist weiter ein Verfahren zur Herstellung der Verbindungen der Formel I, welches darin besteht, dass man

a) ein Amin der Formel II:

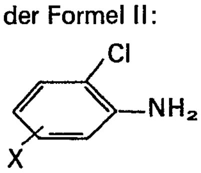

(II)

worin X die angegebene Bedeutung hat, mit einem 1-Aroyl-2-imidazolidinon der Formel III:

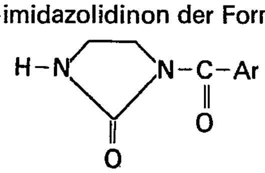

(III)

in der Ar die oben angegebene Bedeutung hat, in Gegenwart von mindestens 1 mol Phosphoroxytrichlorid bei Temperaturen von 20 bis 120°C, oder

b) das aktivierte Derivat einer Arylcarbonsäure der Formel IV:

$$Ar-COOH \quad (IV)$$

worin Ar dasselbe wie oben bedeutet, mit einem 2-Arylamino-2-imidazolin der Formel V:

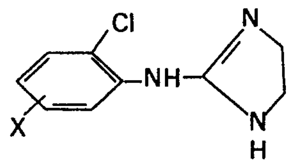

(V)

worin X dasselbe wie oben bedeutet,

umsetzt und die so erhaltenen Verbindungen gegebenenfalls in ihre Salze mit physiologischen Säuren überführt.

Die Umsetzung des Amins II gemäss a mit einem 1-Aroyl-2-imidazolidinon III erfolgt bevorzugt im Molverhältnis 1:1,1 bis 1:1,5, und darüber hinaus zweckmässig in überschüssigem $POCl_3$ als Lösungsmittel, bevorzugt bei Temperaturen von 40 bis 70°C oder Rückflusstemperatur eines gegebenenfalls verwendeten Lösungsmittels. Zweckmässigerweise kann die Reaktion auch in Gegenwart eines inerten organischen Lösungsmittels, z.B. Chlorkohlenwasserstoffen, wie Chloroform oder Tetrachlorkohlenstoff, durchgeführt werden. Es ist ausserdem vorteilhaft, wegen der verhältnismässig leichten Oxidierbarkeit der verwendeten Amine in einer inerten Atmosphäre, beispielsweise unter Stickstoff oder Argon, zu arbeiten.

Nach Beendigung der Umsetzung, die z.B. durch Dünnschichtchromatographie verfolgt werden kann, wird das überschüssige $POCl_3$ durch Destillation entfernt, und nach Neutralisation werden die erhaltenen erfindungsgemässen Verbindungen I gegebenenfalls durch physikalische Methoden, wie beispielsweise Verteilung, Kristallisation, Chromatographie, oder durch chemische Methoden, wie z.B. Bildung von Salzen, Kristallisation und anschliessende Zerlegung in alkalischen Medien, gereinigt. Bei der Reinigung spielt die Art des Anions des Salzes keine Rolle, da es nur darauf ankommt, dass das Salz gut definiert und leicht kristallisierbar ist.

Das überschüssige $POCl_3$ wird vorzugsweise abdestilliert, was zur Schonung des Reaktionsproduktes zweckmässigerweise unter vermindertem Druck erfolgt. Der erhaltene Destillationsrückstand wird in der Kälte mit Wasser, vorzugsweise Eiswasser, versetzt, mit wässerigen alkalischen Lösungen, wie z.B. Natriumhydrogencarbonat-, Natriumcarbonat- oder Natriumhydroxydlösung bis zur deutlich alkalischen Reaktion, alkalisch gemacht und mit einem mit Wasser nicht mischbaren Lösungsmittel, wie Methylenchlorid oder Chloroform, extrahiert. Nach dem Entfernen des Lösungsmittels werden die erfindungsgemässen Substanzen I vorzugsweise durch Kristallisation aus einem inerten organischen Lösungsmittel, wie z.B. Hexan, Cyclohexan, Toluol, Isopropanol oder Acetonitril, gereinigt.

Die als Ausgangsverbindungen verwendeten 1-Aroyl-2-imidazolidinone der Formel III sind noch

nicht beschrieben. Sie lassen sich durch Acylierung von Ethylenharnstoff mit Arylcarbonsäureanhydriden oder mit Arylcarbonsäurehalogeniden gegebenenfalls in Gegenwart von säurebindenden Mitteln, wie z.B. Triethylamin, Pyridin oder Antipyrin, in an sich üblicher Weise und in guten Ausbeuten erhalten.

Für das Verfahren b eignen sich als aktivierte Arylcarbonsäurederivate beispielsweise Azolide mit einem heterocyclischen quasiaromatischen Fünfring mit mindestens zwei Stickstoffatomen im Ring, wie z.B. Imidazol, Pyrazol, 1,2,3-Triazol, 1,2,4-Triazol, 1,2,4-Oxdiazol, Tetrazol, Benzimidazol, Benztriazol und deren Substitutionsprodukte.

Von den Azoliden sind die Imidazolide aufgrund ihrer leichten und wohlfeilen Zugänglichkeit besonders bevorzugt. Sie können nach bekannten Verfahren entweder durch Umsetzung von Arylcarbonsäuren mit N,N'-Carbonyldiimidazol bzw. N,N'-Thiocarbonyldiimidazol oder durch Umsetzung von Arylcarbonsäurechloriden mit Imidazol im Molverhältnis 1:2 in praktisch quantitativer Ausbeute hergestellt werden. Ihre weitere Umsetzung mit den Imidazolinen der Formel V geschieht nach den für Transacylierungsreaktionen von Imidazoliden gebräuchlichen Methoden.

Zweckmässig wird so verfahren, dass man die freie Arylcarbonsäure bei Raumtemperatur mit N,N'-Carbonyldiimidazol im Molverhältnis 1:1 in einem inerten Lösungsmittel umsetzt und nach beendeter $CO_2$-Entwicklung das Imidazolin V hinzugibt. Die Reaktionszeit ist von der Reaktivität des Imidazolids abhängig. In der Regel ist die Reaktion jedoch bei Raumtemperatur nach 1 bis 2 h beendet, jedoch kann gegebenenfalls auch erwärmt werden, maximal bis zum Siedepunkt des verwendeten Lösungsmittels. Als Lösungsmittel eignen sich z.B. Methylenchlorid, Chloroform, Benzol, Toluol, Xylol, Tetrahydrofuran, Dioxan, N,N'-Dimethylacetamid, N-Methylpyrrolidinon-2, 1,3-Dimethyl-2-imidazolidinon, 1,3-Dimethyl-3,4,5,6-tetrahydro-2-[1H]-pyrimidinon, N,N'-Dimethylformamid, Hexamethylphosphorsäuretriamid, Dimethylsulfoxid und Acetonitril oder Mischungen daraus.

Die Aufarbeitung erfolgt auf herkömmliche Weise in Abhängigkeit vom verwendeten Lösungsmittel. Entweder können die erfindungsgemässen Substanzen durch direkte Zugabe von wässerigen alkalischen Lösungen zur Reaktionsmischung, wie z.B. Natriumhydrogencarbonat-, Natriumcarbonat- oder Natriumhydroxydlösung bis zu einem pH=8 kristallin ausgefällt werden, oder die Isolierung erfolgt gleichartig nach vorherigem Entfernen des Lösungsmittels und gegebenenfalls Extraktion mit einem Lösungsmittel.

Es sei darauf hingewiesen, dass die erfindungsgemässen Verbindungen der allgemeinen Formel I, ebenso wie die 2-Arylamino-2-imidazoline der Formel IV, teilweise oder vollständig in einer zweiten tautomeren Form entsprechend der Formel Ia vorliegen können:

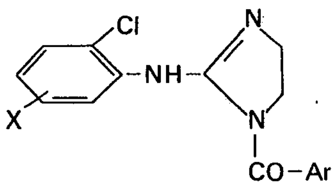

(I)

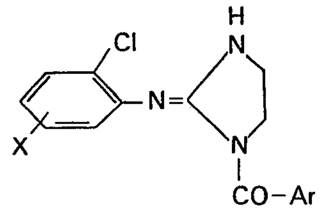

(Ia)

Je nach den Verfahrensbedingungen und den Ausgangsmaterialien erhält man das Endprodukt entweder als die freie Base oder als deren Säureadditionssalz.

So können basische, neutrale oder gemischte Salze sowie Mono-, Sesqui- oder Polyhydrate erhalten werden. Die Säureadditionssalze der neuen Verbindungen können in an sich bekannter Weise in die freie Base überführt werden, indem man basische Mittel, wie Alkali, oder Ionenaustauscher anwendet. Andererseits können die erhaltenen freien Basen mit organischen oder anorganischen Säuren Salze bilden. Bei der Herstellung von Säureadditionssalzen werden vorzugsweise solche Säuren verwendet, wie sie in „J. Pharm. Sci.", *66*, 1-16 (1977) beschrieben werden und die geeignete, therapeutisch verträgliche Salze bilden. Solche Säuren sind beispielsweise Halogenwasserstoffsäuren, Schwefel-, Phosphor-, Salpeter- und Perchlorsäure, aliphatische, alicyclische, aromatische, heterocyclische Carbon- oder Sulfonsäure, wie Ameisen-, Propion-, Bernstein-, Glycol-, Milch-, Äpfel-, Wein-, Zitronen-, Ascorbin-, Malein-, Fumar-, Pyruv-, Phenylessig-, Benzoe-, p-Aminobenzoe-, Anthranil-, p-Hydroxybenzoe-, Salicyl- oder p-Aminosalicylsäure, Embon-, Methansulfon-, Ethansulfon-, Hydroxyethansulfon-, Ethylensulfon-, Halogenbenzolsulfon-, Toluolsulfon-, Naphthylsulfon- oder Sulfanilsäure, Methionin, Tryptophan, Lysin oder Arginin.

Diese oder andere Salze der neuen Verbindungen, wie beispielsweise Picrate, können zur Reinigung der erhaltenen freien Basen dienen. Hierzu können Salze dieser Basen gebildet, von der Lösung abgetrennt und dann die freie Base aus einer neuen Salzlösung in einem reineren Zustand zurückgewonnen werden.

Die Ausgangsmaterialien sind bekannt oder können, falls sie neu sein sollten, nach an sich bekannten Verfahren erhalten werden.

Die erfindungsgemässen Verbindungen und ihre physiologisch verträglichen Säureadditionssalze sind als Pharmaka mit blutdrucksenkender und vergleichsweise geringer sedativer Wirkung zur Behandlung der Hypertonie geeignet.

Zur Untersuchung der pharmakologischen Eigenschaften wurden folgende Methoden verwendet:

*1. Antihypertensive Wirkung*

Die Substanzen werden Gruppen von 4 bis 8 männlichen, spontan hypertonen Okamoto-Ratten (SH-Ratten) oral appliziert. Vor und 2 h nach der Applikation wird der systolische Blutdruck am Rattenschwanz mit Hilfe von Piezokristallaufnehmern gemessen.

Als ED 20% wird unter Berücksichtigung der Werte unbehandelter Kontrolltiere aus den Dosiswirkungsbeziehungen die Dosis bestimmt, welche den systolischen Druck um 20% senkt.

*2. Narkoseverlängernde Wirkung an der Ratte*

Die Substanzen werden Gruppen von 5 weiblichen Sprague-Dawley-Ratten 2 h vor i.p. Applikation von 215 mg/kg Chloralhydrat oral verabreicht. Gemessen wird die Zeit des Ausfalls des Umkehrreflexes. Als ED 50% wird die Dosis ermittelt, welche die Narkosedauer im Vergleich zu nicht vorbehandelten Kontrolltieren um 50% verlängert.

*3. Akute Toxizität*

Zur Bestimmung der akuten Toxizität (LD 50) werden die Substanzen Gruppen von 10 männlichen Sprague-Dawley-Ratten von 150 bis 320 g oral appliziert. Die Beobachtungsdauer beträgt 1 Woche.

Als Referenzsubstanzen dienten die bekannten Antihypertonika Clonidin und Benclonidin.

Die erfindungsgemässen Verbindungen (vgl. Tabelle 1) wirken an spontan hypertonen Ratten bei der für die therapeutische Anwendung primär wichtigen oralen Applikation ebenso stark antihypertensiv wie das bekannte Antihypertonikum Clonidin. Im Vergleich zu Benclonidin wirken sie bereits in 9,8- bis 10,6fach (vgl. Tabelle 1) kleineren Dosen antihypertensiv.

Eine unerwünschte Nebenwirkung des Clonidin ist die zentral dämpfende (sedative) Wirkung, die am Modell der Verlängerung der Chloralhydratnarkose getestet wurde. Die für die Narkoseverlängerung notwendigen Dosen sind 1,3- und 5,4mal höher als die zur Blutdrucksenkung. Danach ist das Verhältnis der Dosen aus der sedativen und der antihypertensiven Wirkung insbesondere bei dem der Substanzen des Beispiels 6 deutlich günstiger als bei Clonidin und Benclonidin.

*Tabelle 1*

| Beispiel Nr. | Antihypertensive Wirkung[1] ED 20% | | Narkoseverlängernde Wirkung[2] ED 50% | | LD 50[2] | Q[4] |
|---|---|---|---|---|---|---|
| | (mg/kg) | R.W.[3] | (mg/kg) | R.W.[3] | (mg/kg) | |
| 1 | 0,0786 | 1,05 | 0,1 | 0,57 | 80,0 | 1,27 |
| 6 | 0,0856 | 0,97 | 0,464 | 0,12 | 44,0 | 5,42 |
| Clonidin | 0,0829 | 1,00 | 0,0565 | 1,00 | 67,3 | 0,68 |
| Benclonidin | 0,836 | 0,10 | 0,948 | 0,06 | 68,1 | 1,13 |

[1] SH-Ratte, Appl.: *per os*
[2] Ratte, Appl.: *per os*
[3] R.W. = Relative Wirksamkeit

$$^4\,Q = \frac{\text{ED 50\% Narkoseverlängerung}}{\text{ED 20\% Blutdrucksenkung}}$$

Darüber hinaus eignen sich die neuen Substanzen zur Behandlung von Migräne.

Weiterhin eignen sich die erfindungsgemässen Substanzen aufgrund ihrer Wirkung auf Diastolendauer und Herzfrequenz als Pharmaka zur Behandlung der koronaren Herzkrankheit.

Die erfindungsgemässen Verbindungen können in üblicher Weise oral oder parenteral (subkutan, intravenös, intramuskulär) verabfolgt werden.

Die neuen Verbindungen können in den gebräuchlichen galenischen Applikationsformen fest oder flüssig angewendet werden, z.B. als Tabletten, Kapseln, Pulver, Granulate, Dragées, Suppositorien oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fliessreguliermitteln, Weichmachern, Netzmitteln, Dispergiermitteln, Emulgatoren, Lösungsmitteln, Retardierungsmitteln und/oder Antioxidantien verarbeitet werden. Die so erhaltenen Wirkstoffe enthalten den Wirkstoff normalerweise in einer Menge von 0,1 bis 99 Gew.-%.

Die folgenden Beispiele erläutern die Erfindung:

*Beispiel 1:*

5,5 g (44,7 mmol) Nicotinsäure werden in 800 ml absolutem Tetrahydrofuran gelöst, 7,3 g (45 mmol) N,N'-Carbonyldiimidazol zugegeben und 45 min bei Raumtemperatur gerührt. Unter weiterem Rühren tropft man eine Lösung von 10,3 g (44,7 mmol) 2-(2',6'-Dichlorphenylamino)-2-imidazolin in 200 ml absolutem Tetrahydrofuran zu und lässt über Nacht bei Raumtemperatur stehen. Die Reaktionslösung wird am Rotationsverdampfer vom Lösungsmittel befreit, der Rückstand mit 200 ml einer 0,5%igen Natriumhydrogencarbonatlösung versetzt, das ausfallende Produkt abgenutscht und mit Wasser gewaschen. Nach dem Trocknen wird das Rohprodukt aus Isopropanol umkristallisiert. Man erhält 7,2 g 1-Nicotinoyl-2-(2',6'-dichlorphenylamino)-2-imidazolin, Fp. 173-174°C.

Nach dem in Beispiel 1 beschriebenen Verfahren wurden die folgenden Verbindungen erhalten:

*Beispiel 2:*

Aus Furan-2-carbonsäure und 2-(2',6'-Dichlorphenylamino)-2-imidazolin:

1-(2-Furoyl)-2-(2',6'-dichlorphenylamino)-2-imidazolinhydrat, Fp. 200,5-201,5°C.

*Beispiel 3:*

Aus Nicotinsäure und 2-(2',3'-Dichlorphenylamino)-2-imidazolin:

1-Nicotinoyl-2-(2',3'-dichlorphenylamino)-2-imidazolin, Fp. 173-174,5°C (enthält 0,5 mol Kristallisopropanol).

*Beispiel 4:*

Aus Isonicotinsäure und 2-(2',3'-Dichlorphenylamino)-2-imidazolin:

1-Isonicotinoyl-2-(2',3'-dichlorphenylamino)-2-imidazolin, Fp. 152-154°C.

*Beispiel 5:*

2,7 g (21,0 mmol) Isonicotinsäure gibt man zu 800 ml absolutem Tetrahydrofuran, setzt 4,2 g (25,9 mmol) N,N'-Carbonyldiimidazol zu und kocht 2 h am Rückfluss. Nach dem Abkühlen wird eine Lösung von 5,0 g (21,7 mmol) 2-(2',6'-Dichlorphenylamino)-2-imidazolin in 100 ml absolutem Tetrahydrofuran zugegeben und 50 h bei Raumtemperatur gerührt. Anschliessend wird am Rotationsverdampfer vom Lösungsmittel befreit, der ölige Rückstand in 500 ml Tetrahydrofuran aufgenommen und in 1,5 l 0,1%iger Natriumhydrogencarbonatlösung unter Rühren eingetropft. Das erhaltene Kristallisat (6,5 g) wird je einmal aus Isopropanol/Petrolether 100:140 und aus reinem Isopropanol umkristallisiert. Man erhält 3,0 g reines 1-Isonicotinoyl-2-(2',6'-dichlorphenylamino)-2-imidazolin vom Fp. 82,5-83,5°C (enthält 0,5 mol Kristallisopropanol).

*Beispiel 6:*

2,85 g (16,5 mmol) Chinolin-2-carbonsäure werden in 200 ml absolutem Tetrahydrofuran gelöst, 2,7 g (16,5 mmol) N,N'-Carbonyldiimidazol zugegeben und 45 min bei Raumtemperatur gerührt. Anschliessend tropft man eine Lösung von 3,75 g (16,5 mmol) 2-(2',6'-Dichlorphenylamino)-2-imidazolin in 75 ml absolutem Tetrahydrofuran zu und rührt über Nacht. Die Reaktionslösung wird am Rotationsverdampfer vom Lösungsmittel befreit und der ölige Rückstand in 200 ml einer 0,5%igen Natriumhydrogencarbonatlösung eingetragen. Das kristalline Rohprodukt (5,2 g) wird abgenutscht, aus N,N-Dimethylacetamid/Isopropanol umkristallisiert und mit kaltem Isopropanol gewaschen. Man erhält

3,3 g 1-(2-Chinolinoyl)-2-(2',6'-dichlorphenylamino)-2-imidazolin, Fp. 197-198°C.

*Beispiel 7:*

1,90 g (11 mmol) Chinolin-2-carbonsäure werden in 15 ml absolutem N,N-Dimethylacetamid und 80 ml absolutem Tetrahydrofuran gelöst, 3,49 g (21,5 mmol) N,N'-Carbonyldiimidazol zugesetzt, 45 min bei Raumtemperatur gerührt und eine Lösung von 2,53 g (11 mmol) 2-(2',6'-Dichlorphenylamino)-2-imidazolin in 50 ml absolutem Tetrahydrofuran zugetropft. Anschliessend lässt man 16 h bei Raumpemperatur rühren, engt am Rotationsverdampfer ein, nimmt mit ca. 20 ml Tetrahydrofuran auf, filtriert zur Entfernung geringer Mengen unlöslicher Anteile und zur Entfärbung über Aktivkohle und lässt das Filtrat in 500 ml 0,5%iger $NaHCO_3$-Lösung eintropfen. Das ausgefallene Rohprodukt wird abgenutscht (Ausbeute 3,7 g vom Fp. 187-189°C), in wenig Dimethylacetamid gelöst und durch Eintropfen in Isopropanol umkristallisiert. Man erhält 3,0 g reines 1-(2-Chinolinoyl)-2-(2',6'-dichlorphenylamino)-2-imidazolin vom Fp. 197-198°C, das mit dem nach Beispiel 6 erhaltenen Produkt identisch ist.

*Beispiel 8:*

Nach dem in Beispiel 5 beschriebenen Verfahren erhält man bei Ersatz des N,N-Dimethylacetamids als Co-Lösungsmittel durch die gleiche Menge N,N-Dimethylformamid 2,8 g reines 1-(2-Chinolinoyl)-2-(2',6'-dichlorphenylamino)-2-imidazolin vom Fp. 197-198°C, das mit dem nach Beispiel 6 erhaltenen Produkt identisch ist.

*Beispiel 9:*

Nach dem in Beispiel 5 beschriebenen Verfahren erhält man bei Ersatz des N,N-Dimethylacetamids als Co-Lösungsmittel durch die gleiche Menge N-Methylpyrrolidon-2 2,9 g reines 1-(2-Chinolinoyl)-2-(2',6'-dichlorphenylamino)-2-imidazolin vom Fp. 197-198°C, das mit dem nach Beispiel 6 erhaltenen Produkt identisch ist.

*Beispiel 10:*

Nach dem in Beispiel 7 beschriebenen Verfahren erhält man aus 6-Fluorchinolin-2-carbonsäure und 2-(2',6'-Dichlorphenylamino)-2-imidazolin:

1-(6-Fluor-2-chinolinoyl)-2-(2',6'-dichlorphenylamino)-2-imidazolin, Fp. 183-184,5°C (kristallisiert mit 1 mol THF).

*Beispiel 11:*

4,15 g (24 mmol) Nicotinoylimidazolid und 4,6 g (20 mmol) 2-(2',6'-Dichlorphenylamino)-2-imidazolin werden 3 h in 100 ml absolutem Toluol am Rückfluss gekocht. Anschliessend wird am Rotationsverdampfer zur Trockne eingeengt und der Rückstand wie in Beispiel 5 beschrieben aufgearbeitet. Man erhält 2,9 g reines 1-Nicotinoyl-2-(2',6'-dichlorphenylamino)-2-imidazolin vom Fp. 172,5-173,5°C, das mit dem gemäss Beispiel 1 erhaltenen Produkt identisch ist.

*Beispiel 12:*

Nach dem in Beispiel 9 beschriebenen Verfahren erhält man aus 5,36 g (24 mmol) 2-Chinolinoylimidazolid und 4,6 g (20 mmol) 2-(2',6'-Dichlorphenylamino)-2-imidazolin 6,5 g reines 1-(2-Chinolinoyl)-2-(2',6'-dichlorphenyl-amino)-2-imidazolin vom Fp. 193,5-196°C, das mit dem nach Beispiel 6 erhaltenen Produkt identisch ist.

*Beispiel 13:*

3,40 g (50 mmol) Imidazol werden in 25 ml absolutem Tetrahydrofuran gelöst und eine Lösung von 3,54 g (25 mmol) Nicotinsäurechlorid in 25 ml absolutem Tetrahydrofuran bei Raumtemperatur zugetropft und weitere 2 h gerührt. Dann wird vom ausgefallenen Imidazolhydrochlorid abgesaugt, der Filterrückstand mit wenig wasserfreiem Tetrahydrofuran gewaschen und die vereinigten Filtrate zu einer Lösung von 4,6 g (20 mmol) 2-(2',6'-Dichlorphenylamino)-2-imidazolin in 25 ml absolutem Tetrahydrofuran gegeben und über Nacht gerührt. Anschliessend wird das Lösungsmittel unter vermindertem Druck am Rotationsverdampfer abgezogen und der Rückstand zweimal aus Isopropanol umkristallisiert. Man erhält 4,8 g reines 1-Nicotinoyl-2-(2',6'-dichlorphenylamino)-2-imidazolin vom Fp. 172,5-174°C, das mit dem in Beispiel 1 beschriebenen Produkt identisch ist.

*Beispiel 14:*

1,9 g (10 mmol) 1-Nicotinoylimidazolin-2-on (Fp. 191,5-193°C) und 1,46 g (9 mmol) 2,6-Dichloranilin werden in 30 ml Phosphoroxychlorid 50 h bei 50°C gerührt. Das überschüssige Phosphoroxychlorid wird unter vermindertem Druck am Rotationsverdampfer abgezogen. Der Rückstand wird mit Eis/Wasser versetzt und 30 min geschüttelt, mit 40%iger Natronlauge alkalisch gestellt und dreimal mit Chloroform extrahiert. Die vereinigten organischen Phasen werden einmal mit 1 N-Natronlauge und anschliessend mit Wasser neutral gewaschen, über Natriumsulfat getrocknet und unter vermindertem Druck am Rotationsverdampfer zur Trockne eingeengt. Der Rückstand wird mit heissem Petrolether digeriert und der kristalline Rückstand aus Isopropanol umkristallisiert. Man erhält 0,87 g reines 1-Nicotinoyl-2-(2',6'-dichlorphenylamino)-2-imidazolin vom Fp. 171-173°C, das mit dem nach Beispiel 1 erhaltenen Produkt identisch ist.

*Beispiel 15:*

Nach dem in Beispiel 14 beschriebenen Verfahren erhält man aus 1-(2-Chinolinoyl)imidazolin-2-on und 2,6-Dichloranilin in Gegenwart eines Überschusses von Phosphoroxychlorid 1-(2-Chinolinoyl)-2-(2',6'-dichlorphenylamino)-2-imidazolin vom Fp. 196-197,5°C, das mit dem nach Beispiel 6 erhaltenen Produkt identisch ist.

*Beispiel 16:*

Tabletten zu 0,1 mg 1-(2-Chinolinoyl-2-(2',6'-dichlorphenylamino)-2-imidazolin:

| | |
|---|---|
| 1-(2-Chinolinoyl-2-(2',6'-dichlorphenylamino)-2-imidazolin | 0,1 mg |
| Milchzucker | 54,9 mg |
| Maisstärke | 30 mg |
| Calciumhydrogenphosphatdihydrat | 15 mg |
| lösliche Stärke | 3 mg |
| Magnesiumstearat | 2 mg |
| kolloidale Kieselsäure | 5 mg |
| | 110 mg |

*Herstellung:* Der Wirkstoff wird mit einem Teil der Hilfsstoffe vermischt, intensiv mit einer wässerigen Lösung der löslichen Stärke durchgeknetet und in üblicher Weise mit Hilfe eines Siebes granuliert. Das Granulat wird mit dem Rest der Hilfsstoffe vermischt und zu Tabletten von je 110 mg Gewicht verpresst. Nach dem gleichen Verfahren lassen sich Dragéekerne herstellen, die dann in üblicher Weise mit Hilfe von Zucker, Talkum und Gummiarabikum dragiert werden können. Das fertig abgemischte Granulat kann auch direkt in Steckkapseln abgefüllt werden.

*Beispiel 17:*

Ampullen zu 0,025 mg 1-Nicotinoyl-2(2',6'-dichlorphenylamino)-2-imidazolin:

| | |
|---|---|
| 1-Nicotinoyl-2-(2',6'-dichlorphenylamino)-2-imidazolin | 0,025 mg |
| Natriumchlorid | 18 mg |
| bidest. Wasser | *ad* 2,0 ml |

*Herstellung:* Der Wirkstoff und das Natriumchlorid werden in Wasser gelöst, die Lösung steril filtriert und unter Stickstoff in Glasampullen zu 2 ml abgefüllt.

*Beispiel 18:*

Tropfen zu 0,1 mg/1 ml (=20 Tropfen) 1-Nicotinoyl-2-(2',6'-dichlorphenylamino)-2-imidazolin:

| | |
|---|---|
| 1-Nicotinoyl-2-(2',6'-dichlorphenylamino)-2-imidazolin | 0,01 g |
| p-Hydroxybenzoesäuremethylester | 0,07 g |
| p-Hydroxybenzoesäurepropylester | 0,03 g |
| Geschmackskorrigentien | 2,0 g |
| Ethanol | 20,0 g |
| entmineralisiertes Wasser | *ad* 100 ml |

*Herstellung:* Die p-Hydroxybenzoesäureester werden in Ethanol gelöst und die alkohollöslichen Geschmackskorrigentien (z.B. Natriumcyclamat) in einem Teil des Wassers gelöst, die beiden Lösungen vereinigt, mit Wasser *ad* 100 ml aufgefüllt, anschliessend klar filtriert und in Tropfflaschen abgefüllt.

*Beispiel 19:*

Suppositorien zu 0,4 mg 1-(2-Chinolinoyl)-2-(2',6'-dichlorphenylamino)-2-imidazolin:

| | |
|---|---|
| 1-(2-Chinolinoyl-2-(2',6'-dichlorphenylamino)-2-imidazolin | 0,4 mg |
| Milchzucker | 244,6 mg |
| Zäpfchenmasse (z.B. Witepsol H 19 und Witepsol W 45) | *ad* 1,7 g |

*Herstellung:* Die Zäpfchenmasse wird geschmolzen. Bei 38°C wird die zusammen mit dem Milchzucker vermahlene Wirksubstanz in der Schmelze homogen dispergiert, auf 35°C abgekühlt und in vorgekühlte Suppositorienformen ausgegossen. Zäpfchengewicht 1,7 g.

**Patentansprüche** für die Vertragsstaaten BE, CH, DE, FR, GB, IT, LI, LU, NL, SE

1. 1-Aroyl-2-phenylamino-2-imidazoline der allgemeinen Formel (I):

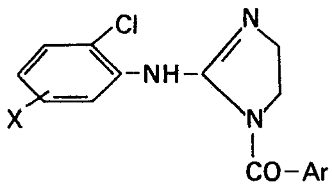

(I)

worin

X ein Chloratom in der 3- oder 6-Stellung des Phenylringes, und

Ar einen 2-Chinolinyl-, 6-Fluor-2-chinolinyl-, 2-Furyl-, 3-Pyridyl- oder 4-Pyridylrest darstellen, sowie deren Salze mit physiologisch verträglichen Säuren.

2. 1-Nicotinoyl-2-(2,6-dichlorphenylamino)-2-imidazolin.

3. 1-(2-Chinolinoyl)-2-(2,6-dichlorphenyl-amino)-2-imidazolin.

4. Verfahren zur Herstellung der Verbindungen der Formel (I) gemäss Anspruch 1, dadurch gekennzeichnet, dass man:

a) ein Amin der Formel (II):

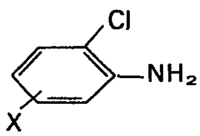

(II)

in der X die oben angegebenen Bedeutungen hat, mit einem 1-Aroyl-2-imidazolidinon der Formel (III):

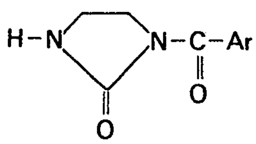

(III)

in der Ar die oben angegebene Bedeutung hat, in Gegenwart von mindestens 1 mol Phosphoroxytrichlorid bei Temperaturen von 20 bis 120° C, oder

b) das aktivierte Derivat einer Arylcarbonsäure der Formel (IV):

Ar−COOH (IV)

worin Ar dasselbe wie oben bedeutet, mit einem 2-Arylamino-2-imidazolin der Formel (V):

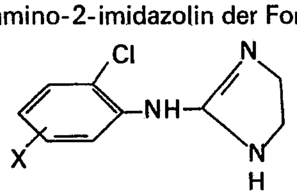

(V)

worin X dasselbe wie oben bedeutet, umsetzt und die so erhaltenen Verbindungen gegebenenfalls in ihre Salze mit physiologischen Säuren überführt.

5. Arzneimittel, enthaltend eine Verbindung gemäss Anspruch 1.

6. Verbindung gemäss Anspruch 1 zur Verwendung bei der Bekämpfung der Hypertonie.

7. Verbindung gemäss Anspruch 1 zur Verwendung bei der Bekämpfung von Migräne.

8. Verbindung gemäss Anspruch 1 zur Verwendung bei der Bekämpfung der koronaren Herzkrankheit.

**Patentansprüche** für den Vertragsstaat: AT

1. Verfahren zur Herstellung von 1-Aroyl-2-phenylamino-2-imidazolinen der allgemeinen Formel (I):

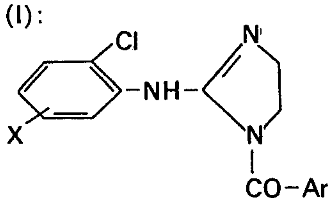

(I)

worin

X ein Chloratom in der 3- oder 6-Stellung des Phenylringes, und

Ar einen 2-Chinolinyl-, 6-Fluor-2-chinolinyl-, 2-Furyl-, 3-Pyridyl- oder 4-Pyridylrest darstellen, sowie von deren Salzen mit physiologisch verträglichen Säuren, dadurch gekennzeichnet, dass man:

a) ein Amin der Formel (II):

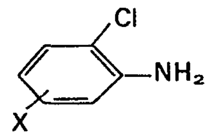

(II)

in der X die oben angegebenen Bedeutungen hat, mit einem 1-Aroyl-2-imidazolidinon der Formel (III):

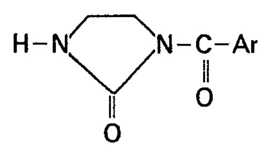

(III)

in der Ar die oben angegebene Bedeutung hat, in Gegenwart von mindestens 1 mol Phosphoroxytrichlorid bei Temperaturen von 20 bis 120° C, oder

b) das aktivierte Derivat einer Arylcarbonsäure der Formel (IV):

Ar-COOH (IV)

worin Ar dasselbe wie oben bedeutet, mit einem 2-Arylamino-2-imidazolin der Formel (V):

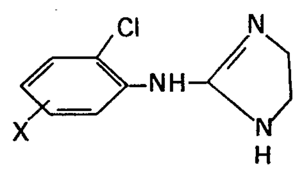

(V)

worin X dasselbe wie oben bedeutet, umsetzt und die so erhaltenen Verbindungen gegebenenfalls in ihre Salze mit physiologischen Säuren überführt.

2. Verfahren gemäss Anspruch 1, dadurch gekennzeichnet, dass man 1-(2-Nicotinoyl)-2-(2,6-dichlorphenylamino)-2-imidazolin herstellt.

3. Verfahren gemäss Anspruch 1, dadurch gekennzeichnet, dass man 1-(2-Chinolinoyl)-2-(2,6-dichlorphenylamino)-2-imidazolin herstellt.

**Claims** for the contracting States BE, CH, DE, FR, GB, IT, LI, LU, NL, SE

1. 1-Aroyl-2-phenylamino-2-imidazoline of the general formula (I)—

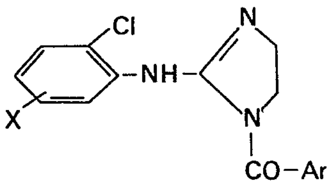

(I)

where

X is chlorine in the 3- or 6-position of the phenyl ring, and

Ar is quinolin-2-yl, 6-fluoroquinolin-2-yl, fur-2-yl, pyrid-3-yl or pyrid-4-yl, and its salts with physiologically tolerated acids.

2. 1-Nicotinoyl-2-(2,6-dichlorophenylamino)-2-imidazoline.

3. 1-(Quinolin-2-oyl)-2-(2,6-dichlorophenyl-amino)-2-imidazoline.

4. A process for the preparation of a compound of the formula (I) as claimed in Claim 1, wherein—

(*a*) an amide of the formula (II)—

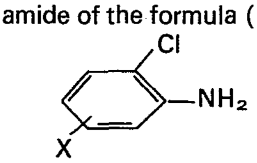

(II)

where X has the above meaning, is reacted with a 1-aroyl-2-imidazolidinone of the formula (III)—

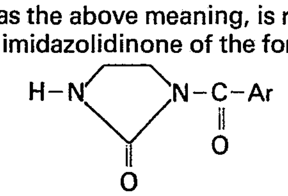

(III)

where Ar has the above meanings, in the presence of not less than 1 mol of phosphorus oxytrichloride, at from 20 to 120°C, or

(*b*) the activated derivative of an alkylcarboxylic acid of the formula (IV)—

Ar−COOH (IV)

where Ar has the same meanings as above, is reacted with a 2-arylamino-2-imidazoline of the formula (V)—

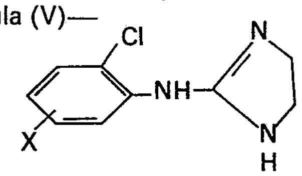

(V)

where X has the same meaning as above, and, if desired, the resulting compound is converted into a salt with a physiologically tolerated acid.

5. A drug containing a compound as claimed in Claim 1.

6. A compound as claimed in Claim 1 for use in the treatment of hypertonia.

7. A compound as claimed in Claim 1 for use in the treatment of migraine.

8. A compound as claimed in Claim 1 for use in the treatment of coronary heart disease.

**Claims** for the contracting State: AT

1. A process for the preparation of a 1-aroyl-2-phenylamino-2-imidazoline of the general formula (I)—

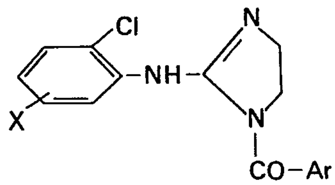

(I)

where

X is chlorine in the 3- or 6-position of the phenyl ring, and

Ar is quinolin-2-yl, 6-fluoroquinolin-2-yl, fur-2-yl, pyrid-3-yl or pyrid-4-yl,

and its salts with physiologically tolerated acids, wherein—

(*a*) an amine of the formula (II)—

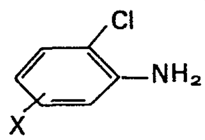

(II)

where X has the above meaning, is reacted with a 1-aroyl-2-imidazolidinone of the formula (III)—

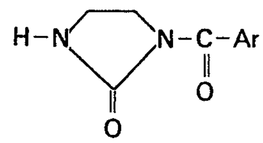

(III)

where Ar has the above meanings, in the presence of not less than 1 mol of phosphorus oxytrichloride, at from 20 to 120°C, or

(*b*) the activated derivative of an alkylcarboxylic acid of the formula (IV)—

Ar−COOH (IV)

where Ar has the same meanings as above, is reacted with a 2-arylamino-2-imidazoline of the formula (V)—

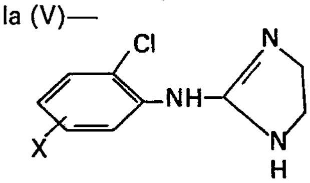

(V)

where X has the same meaning as above, and, if desired, the resulting compound is converted into a salt with a physiologically tolerated acid.

2. A process as claimed in Claim 1, wherein 1-(2-nicotinoyl)-2-(2,6-dichlorophenylamino)-2-imidazoline is prepared.

3. A process as claimed in Claim 1, wherein 1-(quinolin-2-oyl)-2-(2,6-dichlorophenylamino)-2-imidazoline is prepared.

**Revendications** pour les Etats contractants BE, CH, DE, FR, GB, IT, LI, LU, NL, SE

1. 1-Aroyl-2-phénylamino-2-imidazolines de la formule générale (I):

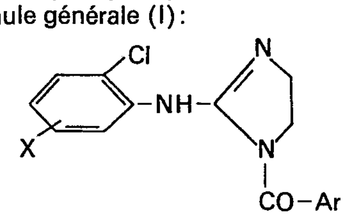

(I)

dans laquelle

X représente un atome de chlore en position 3 ou 6 du noyau phényle, et

Ar représente un reste 2-quinolinyle, 6-fluor-2-quinolinyle, 2-furyle, 3-pyridyle ou 4-pyridyle, ainsi que leurs sels d'acides acceptables physiologiquement.

2. 1-Nicotinoyl-2-(2,6-dichlorophénylamino)-2-imidazoline.

3. 1-(2-Quinolinoyl)-2-(2,6-dichlorophénylamino)-2-imidazoline.

4. Procédé de préparation des composés de formule (I) selon la revendication 1, caractérisé par le fait que l'on fait réagir:

a) une amine de formule (II):

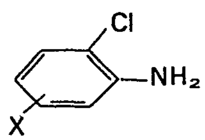

(II)

dans laquelle X a les significations indiquées ci-dessus, avec une 1-aroyl-2-imidazolidinone de formule (III):

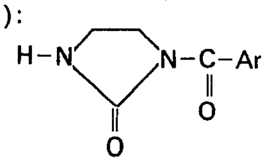

(III)

dans laquelle Ar a la signification indiquée ci-dessus, en présence d'au moins 1 mol d'oxytrichlorure de phosphore, à des températures de 20 à 120°C, ou

b) le dérivé activé d'un acide arylcarboxylique de formule (IV):

Ar−COOH (IV)

dans laquelle Ar a la même signification que ci-dessus, avec une 2-arylamino-2-imidazoline de formule (V):

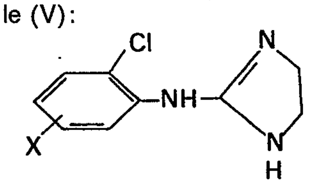

(V)

où X a la même signification que ci-dessus, et les composés ainsi obtenus sont éventuellement transformés en leurs sels d'acides physiologiques.

5. Médicament, contenant un composé selon la revendication 1.

6. Composé selon la revendication 1, pour l'utilisation dans la lutte contre l'hypertonie.

7. Composé selon la revendication 1, pour l'utilisation dans la lutte contre la migraine.

8. Composé selon la revendication 1, pour l'utilisation dans la lutte contre les maladies cardiaques coronariennes.

**Revendications** pour l'Etat contractant: AT

1. Procédé de préparation de 1-aroyl-2-phénylamino-2-imidazolines de la formule générale (I):

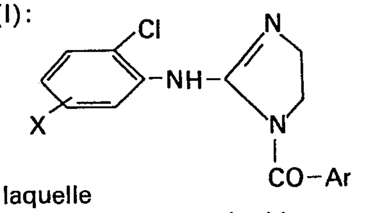

(I)

dans laquelle

X représente un atome de chlore en position 3 ou 6 du noyau phényle, et

Ar représente un reste 2-quinolinyle, 6-fluor-2-quinolinyle, 2-furyle, 3-pyridyle ou 4-pyridyle, ainsi que leurs sels d'acides acceptables physiologiquement, caractérisé par le fait que l'on fait réagir:

a) une amine de formule (II):

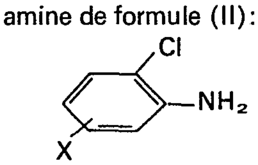

(II)

dans laquelle X a les significations indiquées ci-dessus, avec une 1-aroyl-2-imidazolidinone de formule (III):

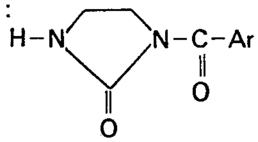

(III)

dans laquelle Ar a la signification indiquée ci-dessus, en présence d'au moins 1 mol d'oxytrichlorure de phosphore, à des températures de 20 à 120°C, ou

b) le dérivé activé d'un acide arylcarboxylique de formule (IV):

Ar−COOH (IV)

dans laquelle Ar a la même signification que ci-dessus, avec une 2-arylamino-2-imidazoline de formule (V):

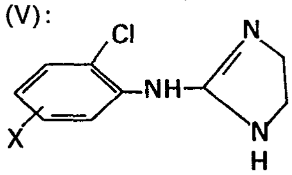

(V)

où X a la même signification que ci-dessus, et les composés ainsi obtenus sont éventuellement transformés en leurs sels d'acides physiologiques.

2. Procédé selon la revendication 1, caractérisé par le fait qu'on prépare la 1-nicotinoyl-2-(2,6-dichlorophénylamino)-2-imidazoline.

3. Procédé selon la revendication 1, caractérisé par le fait qu'on prépare la 1-(2-quinolinoyl)-2-(2,6-dichlorophénylamino)-2-imidazoline.